# EUROPEAN PATENT APPLICATION

(11) **EP 4 692 297 A2**
(43) Date of publication of application: **11.02.2026**
(21) Application number: 24721696.3
(22) Date of filing: 14.03.2024
(51) Int. Cl.: C12M 1/00, C12M 1/34

(54) **DEVICE AND PROCEDURE FOR ONLINE MEASUREMENT OF BIOMASS CONCENTRATION IN MICROALGAE CULTURES**

(30) Priority: 24.03.2023 ES 202330246
(71) Applicant: Universidad de Almeria, 04120 La Cañada de San Urbano (Almeria) (ES)
(72) Inventor: GONZÁLEZ HERNÁNDEZ, José, 04120 La Cañada de San Urbano (Almería) (ES); GUZMÁN SÁNCHEZ, José Luis, 04120 La Cañada de San Urbano (Almería) (ES); ACIEN FERNÁNDEZ, F. Gabriel, 04120 La Cañada de San Urbano (Almería) (ES)
(74) Representative: Pons IP
(86) International application number: PCT/ES2024/070163
(87) International publication number: WO 2024/200891

(57) **Abstract**

The device comprises an absorbance sensor (3), which works with three wavelengths between 400 and 700 nm, intended to emit and collect light reflected from the culture (1), and a sampling pump (2) to the circulation of the culture (1) through the absorbance sensor (3). For its part, the procedure includes the steps of illuminating the culture (1) with light with three different wavelengths, between 400 and 700 nm, collecting a light reflected from the crop (1), and obtaining the RGB colour coordinates. of the reflected light, obtain the absorbance from the RGB coordinates, perform a regression with absorbance and reference concentration data, and obtain the biomass concentration in the culture (1).

## Description

### OBJECT OF THE INVENTION

The object of the invention is a device for online measurement of biomass concentration in microalgae cultures, including cyanobacteria. The invention falls within the field of microalgae production for any application, especially using large-scale reactors.

More specifically, the invention is related to the online determination of the concentration of microalgae biomass (including cyanobacteria) in cultures carried out in industrial reactors, although it can apply to any other type of laboratory or pilot-scale reactors. The invention makes it possible to continuously determine the concentration of biomass in the culture and thus its production in the reactor, or even whether or not the operating conditions are negatively affecting said productivity.

### BACKGROUND OF THE INVENTION

The production of microalgae (including cyanobacteria) is an industrial activity in continuous growth due to the large number of applications that this type of microorganism presents. Among them, it is worth highlighting the production of food and nutraceuticals, cosmetic products, or feed for livestock and aquaculture. All of these applications require the stable and safe production of high-quality biomass, free of contaminants or inappropriate materials that could negatively affect this type of use.

To carry out this production, different types of reactors, both open and closed, are used, in which an attempt is made to control the operating conditions so that the microalgae cells (including cyanobacteria) are in their most favourable state and grow at the highest as possible. These reactors are installed on large areas of land, on the order of hectares, with the installation of multiple units in the same facility being very common since the maximum size of the reactors is limited for various technological reasons.

Whatever the type of reactor used for the production of microalgae, culture media prepared by enriching water with inorganic nutrients are used so that the microalgae can carry out photosynthesis, producing large amounts of oxygen and for this it is also necessary to provide large amounts of CO₂. Specifically, it has been reported that this type of system can produce up to 100 tn / ha·year of biomass, for which it is necessary to provide 200 tn / ha·year of CO₂, as well as 10 tn / ha·year of nitrogen, and 2 tn / ha·year of phosphorus, in the process also producing 150 tn / ha·year of O₂.

For the photosynthetic process to take place, the culture must receive the largest amount of solar radiation possible, and provide it with the optimal conditions for each species such as pH, dissolved oxygen concentration, temperature, etc. Providing these conditions requires an adequate design of the reactor, but also its adequate operation, which inherently entails energy consumption for mixing and thermosetting, the supply of air for the removal of oxygen and the supply of CO₂ for pH control and contribution of inorganic carbon. These contributions are meaningless if the culture does not have enough light to carry out photosynthesis.

The average light in which the cells are found within the culture is a function of the geometry of the reactor, that is, the amount of energy that it intercepts from the solar radiation that arrives from the sun, but also of the concentration of biomass and attenuation of the light that it causes. Light attenuation is an intrinsic parameter of biomass and can be easily measured by determining the attenuation constant in the laboratory. Likewise, the intercepted solar radiation can be quantified using pyranometer-type online sensors and analogues. However, biomass concentration is very difficult to measure online, with the majority using offline measurements in the laboratory such as the determination of dry weight. This makes an optimal and on-line adjustment of the culture conditions in the reactor not possible, which reduces its productivity.

On the other hand, said productivity is a function of the growth rate of the microorganism and the biomass concentration in the reactor. The growth rate itself is defined as the ratio between the increase in biomass concentration per unit of time concerning the biomass concentration. Having only offline measurements that require long processing times means that it is not possible to know the real productivity of biomass until at least 24 hours after having taken the measurements.

To optimize the operation of the microalgae production reactors, it is necessary to know online both the biomass concentration and the growth speed of the culture and thus its productivity. The coupling of this information with the environmental and operating variables of the reactor would allow the development of models and online optimization tools that would maximize the performance of this type of system.

However, this is not an easy task. Until now, there are only some turbidity sensors available on the market that are only valid for certain solids concentration ranges, whether for concentrated (>1 g/L) or diluted (<0.1 g/L) cultures, but not for any range. of concentrations. Furthermore, these sensors do not differentiate between the solids present in the crop since they only use a narrow range of wavelengths. These sensors present interference due to changes in solar radiation throughout the day and require continuous calibration if changes occur in the state of the culture. Finally, the cost of these sensors is extremely high and they require a lot of maintenance to prevent them from becoming dirty or clogged, leading to false readings.

### DESCRIPTION OF THE INVENTION

The online measurement device for biomass concentration in microalgae cultures, the object of the present invention, is presented as an alternative to the devices of the state of the art and includes a culture sampling pump, with a filtration and self-cleaning system to avoid dirtying the sensor or blocking it. The device also includes an absorption sensor that considers a wide range of wavelengths so it can differentiate between different types of solids, especially microalgae cells (including cyanobacteria). Furthermore, the object of the invention is a procedure for measuring biomass concentration in microalgae cultures that transforms a measured absorption signal into the final biomass concentration of the culture.

The device and procedure allow online measurement of the concentration of microalgae biomass (including cyanobacteria) in microalgae cultures at any scale, from laboratory to closed reactors of 3,000 L and open reactors of up to 100 m ³ in volume.

The device and procedure allow for a continuous, highly sensitive measurement in any range of crop biomass concentrations, which does not require labour for its maintenance and allows it to be coupled with any data acquisition and control system for the optimization of the operation of this type of system.

The proposed invention is robust and scalable and can be applied to any type of reactor, both open and closed. It is independent of the species of microalgae that you want to produce or its state (free or flocculated), since it does not depend on a specific type of microalgae or cyanobacteria but is adaptable to any species. Furthermore, it is a cost-competitive device, simple in its methodology and does not require infrastructure or manpower for its operation.

Specifically, the device of the invention is intended to be linked to a reactor and comprises, first of all, an absorbance sensor that works at three different wavelengths between 400 and 700 nm and that preferably includes a light source, a transmission camera and a sensor of specific wavelengths, selected to guarantee the correct identification of the microalgae biomass as such, differentiating it from other solids that could be present in the culture.

Secondly, the device includes a sampling pump for the circulation of the culture through the measurement sensor, which is previously filtered to avoid the presence of improper materials that could damage the sensor. This pump operates automatically with different cleaning cycles to guarantee a correct measurement of the biomass concentration in the culture.

Connected to the sensor, the device comprises, in one aspect of the invention, a processor and a communications module intended to connect to other elements, such as a screen and keyboard for display and operation, and analog/digital outputs for communication.

Furthermore, in one aspect of the invention, the device comprises a protective casing for the entire assembly that allows the device to be located outdoors, and that allows safe and stable operation thereof, withstanding environmental conditions such as rain and wind, and which protects the mechanical and electrical parts of the device.

The type of culture or application of the microalgae biomass (including cyanobacteria) to which the present invention can be applied is not critical, and it can be used both for the production of biomass for human and/or animal consumption, as well as for any other application such as wastewater treatment or the production of biomaterials including biofuels. Furthermore, the application can be used in any type of reactor, whether open, including thin-layer raceway reactors among others, or closed, such as bubble columns or tubular reactors.

It operates automatically, without the need for manual operation or frequent maintenance, with minimal energy consumption and minimizing its impact on the circulation of the culture in the reactor, which does not impair its correct operation.

Regarding its methodology, the procedure of the invention comprises a first step of illuminating the culture with light using three different wavelengths, between 400 and 700 nm. Next, the light reflected from the culture is read. Afterwards, the colour coordinates of the reflected light are obtained and from these the absorbance is transformed into the biomass concentration, correlating it with previous data.

The main advantages derived from the device and procedure of the present invention are:
- Allows the monitoring of the biomass concentration in microalgae cultures (including cyanobacteria) automatically, eliminating the need for labour and waiting times required by other methodologies such as dry weight.
- Allows online determination of biomass productivity and how it is affected by changes in environmental and operating conditions.
- Can be applied to any type of reactor, both open and closed.
- Allows the increasing the productivity of biomass and facilitates the safety and operation of the subsequent biomass harvesting and processing processes.
- It has been experimentally proven that it allows increasing the productivity of cultures by allowing the reactors to operate under optimal conditions in each circumstance.
- Reduces the danger of washing and allows alerting of unfavourable crop situations as a virtual crop status sensor.

### DESCRIPTION OF THE DRAWINGS

In order to supplement the present description and to facilitate a better understanding of the characteristics of the invention, and following a preferred example of its practical implementation, a set of drawings is attached as an integral part of the said description, wherein, by way of illustration and not as a limitation, the following has been represented:
Figure 1.- Shows a schematic representation of the device.
Figure 2.- Shows a detailed view of the sensor.

### PREFERRED EMBODIMENT OF THE INVENTION

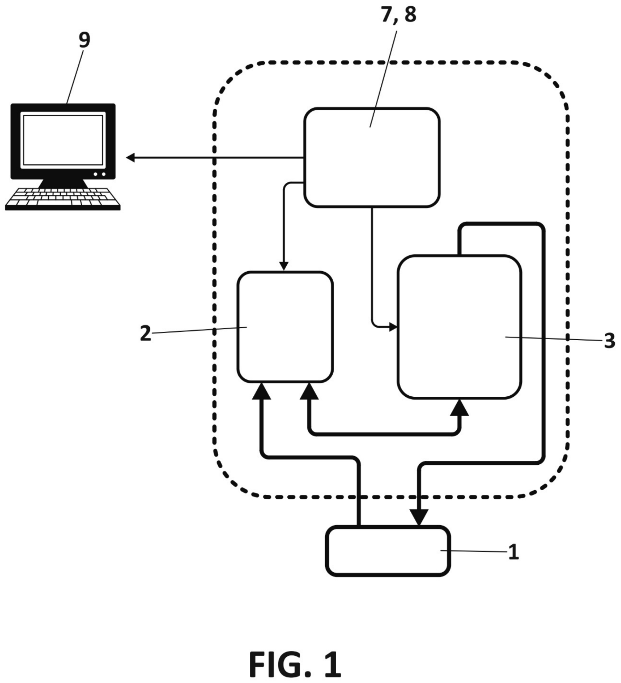

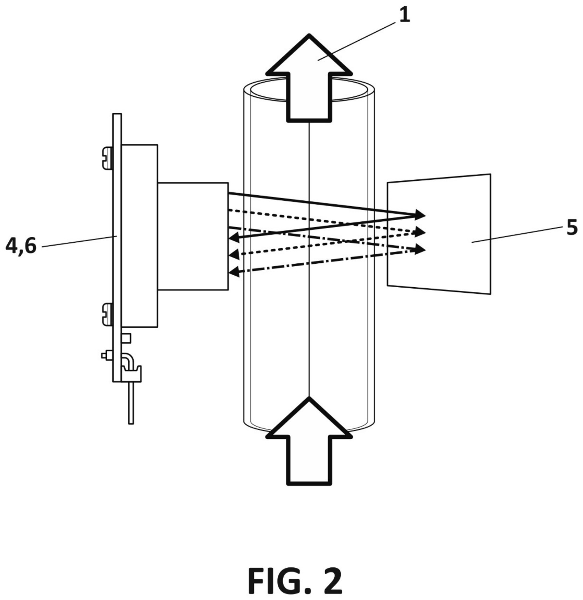

A preferred embodiment of the device and procedure for online measurement of biomass concentration in microalgae cultures is described below, with the help of Figures 1 and 2.

As shown in Figure 1, the device comprises an absorbance sensor (3), intended to obtain data from a culture (1), which works with three wavelengths between 400 and 700 nm, preferably 480, 525 and 610nm. As reflected in detail in Figure 2, the absorbance sensor comprises a light source (4), which emits light at a certain wavelength oriented towards the culture (1), a transmission chamber (5) and a detector (6) of specific wavelengths that receives the light emitted by the light source (4) once it has been reflected by the culture (1). The wavelengths are selected to guarantee the correct identification of the microalgae biomass as such in the culture (1), differentiating it from other solids that may be present.

Secondly, the device comprises a sampling pump (2) for the circulation of the culture (1) through the absorbance sensor (3), which includes a filtering and self-cleaning module, adjustable depending on the specific application (type of microalgae and reactor) to avoid the presence of improper materials that could damage the sensor. Said sampling pump (2) operates automatically with different cleaning cycles to guarantee a correct measurement of the biomass concentration in the culture (1).

Connected to the absorbance sensor (3), the device comprises a processor (7) and a communications module (8) intended to connect to other elements, such as a screen and keyboard for display and operation, and analog/digital outputs for communication with external equipment (9), as appears in figure 1.

The device also includes a protective casing for the mechanical and electrical parts, to protect it from the environment and allow safe and stable operation.

For its part, the method of the invention comprises a first step of illuminating the culture (1) with light using three different wavelengths, between 400 and 700 nm (480, 525 and 610 nm preferably). Next, the light reflected from the culture is read. Then, the colour coordinates of the reflected light, particularly its absorbance, are obtained. Finally, a regression is performed using absorbance and concentration data previously obtained in a calibration stage, using the dry weight technique, and transformed into the biomass concentration, correlating it with previous data.

In one embodiment of the invention, the device is installed in a 100 m ² raceway reactor and is started at 9:00 in the morning. The sample pump (2) pumped 10 mL /min of culture from the reactor recirculated it through the absorbance sensor (3), and then returned to the reactor. In this transit, the absorption of the culture is determined, at different wavelengths, information that is processed for its transformation into the actual measurement of the concentration of microalgae biomass in the culture (1).

In this example, the use of the proposed device makes it possible to monitor with high precision the evolution of the biomass concentration in the culture (1), and thus the change in biomass productivity throughout the day. Furthermore, the integration of this information with a data acquisition system makes it possible to determine the influence of operating conditions on biomass productivity, which has translated into an improvement in the control of said variables and with it an increase in the overall system productivity greater than 35%.

## Claims

1. Online measurement device for biomass concentration of a microalgae culture (1) in a reactor, **characterized in that** it comprises:
- an absorbance sensor (3), which works with three wavelengths between 400 and 700 nm, intended to emit and collect light reflected from the culture (1),
- a sampling pump (2) for the circulation of the culture (1) from the reactor to the absorbance sensor (3), and
- a processor (7) connected to the absorbance sensor (3), configured to obtain the biomass concentration in the culture (1) from the absorbance of the culture (1).

2. The device of claim 1, wherein the absorbance sensor (3) comprises a light source (4), which emits light with three wavelengths between 400 and 700 nm oriented towards the culture (1), a transmission camera (5) and a wavelength detector (6) that receives the light emitted by the light source (4) once it has been reflected by the culture (1).

3. The device of claim 1, wherein the absorbance sensor works with three wavelengths of 480, 525 and 610 nm.

4. The device of claim 1, additionally comprising a protective casing for the absorbance sensor (3), the sampling pump and the processor (7).

5. The device of claim 1, wherein the sampling pump (2) additionally comprises a filtering and self-cleaning module.

6. The device of claim 1, which additionally comprises a communications module (8) connected to the processor (7).

7. Procedure for online measurement of biomass concentration in a microalgae culture (1), which comprises the steps of:
- illuminating the crop (1) with light using three different wavelengths, between 400 and 700 nm,
- collecting a light reflected from the culture (1),
- obtaining the RGB (red, green and blue) coordinates of the reflected light,
- obtaining the absorbance from the RGB coordinates, and
- performing a regression with absorbance and reference concentration data, and obtaining the biomass concentration in the culture (1).

8. The method of claim 7, wherein the wavelengths are 480, 525 and 610 nm.

9. A computer program **characterized in that** it is adapted to carry out the steps of the procedure according to any of claims 7 and 8.

10. A machine-readable storage device **characterized in that** it comprises the computer program according to claim 9.
